# EUROPÄISCHE PATENTSCHRIFT

(11) **EP 3 700 442 B1**
(45) Veröffentlichungstag und Bekanntmachung des Hinweises auf die Patenterteilung: **24.07.2024**
(21) Anmeldenummer: 18799457.9
(22) Anmeldetag: 24.10.2018
(51) Int. Cl.: A61B 17/34, A61M 25/06

(54) **MEHRTEILIGES MEDIZINISCHES WERKZEUG**
MULTI-PART MEDICAL TOOL
OUTIL MÉDICAL EN PLUSIEURS PARTIES

(30) Priorität: 24.10.2017 DE 102017124795
(43) Veröffentlichungstag der Anmeldung: 02.09.2020
(73) Patentinhaber: Meidrix Biomedicals GmbH, 73728 Esslingen (DE)
(72) Erfinder: BECK, Olaf Thorsten, 40724 Hilden (DE); BECK, Petra, 40724 Hilden (DE)
(74) Vertreter: Schrell, Andreas
(86) Internationale Anmeldenummer: PCT/EP2018/079171
(87) Internationale Veröffentlichungsnummer: WO 2019/081585

(56) Entgegenhaltungen:
- EP-B1- 1 054 703
- WO-A1-2006/027549
- WO-A1-2012/103459
- WO-A1-2014/071161
- US-A1- 2014 236 144

## Beschreibung

Die Erfindung betrifft ein mehrteiliges medizinisches Werkzeug zur intrakorporalen Injektion eines Fluids, insbesondere zum Positionieren eines flüssigen und/oder zähflüssigen Kollagens in einem menschlichen Gelenk, insbesondere in einem Hüftgelenk oder in einem Schultergelenk, mit mindestens einer Hohlkanüle, mit mindestens einem in der Hohlkanüle anordenbaren flexiblen Schlauchelement, mit mindestens einem Gehäuseteil und mit mindestens einem Verstellelement, wobei die Hohlkanüle und das Schlauchelement jeweils ein proximales Ende und ein distales Ende aufweisen, wobei die Hohlkanüle - im angeordneten Zustand des Schlauchelementes - das Schlauchelement auf einer Mantellänge zumindest teilweise umhüllt und das proximale Ende des Schlauchelementes außerhalb der Mantellänge einen zumindest teilweise gekrümmten Verlauf ausbildet, wobei die Hohlkanüle relativ zu dem Schlauchelement verschiebbar und/oder bewegbar angeordnet ist, wobei das Verstellelement mit der Hohlkanüle funktional wirksam verbindbar und/oder verbunden ist, und wobei durch das Betätigen des Verstellelementes die Hohlkanüle relativ zum Schlauchelement bewegbar und/oder verschiebbar ist, insbesondere die wirksame Mantellänge einstellbar ist.

Das Hüftgelenk eines Menschen ist wie alle großen Gelenke mit einer zumindest teilweise elastischen Knorpelschicht überzogen. Der Knorpel erlaubt ein schmerzfreies Bewegen des Gelenks und hat auch die Fähigkeit, Stöße und Druck zu dämpfen. Der Knorpel ist teilweise elastisch und enthält aber weder Nerven noch Blutgefäße und ist somit grundsätzlich nicht alleine selbstständig regenerationsfähig. Das Knorpelgewebe (hyaliner Knorpel im engeren Sinne) heilt nach einer Schädigung daher grundsätzlich nicht selbstständig. Ein vorhandener Defekt bleibt bestehen und/oder weitet sich infolge der weiteren mechanischen Belastung bei einer entsprechenden Gelenkbewegung mit der Zeit aus. Die Knorpelschicht, insbesondere an den Hüftgelenken, ist nur wenige Millimeter stark und im Vergleich zu anderen menschlichen Gelenken, zum Beispiel dem Schultergelenk oder dem Kniegelenk, deutlich geringer. Knorpelschäden können beispielsweise Folgen von Verletzungen oder von Überbelastung sein. Hierbei wird der Knorpel meist nur an einer Stelle beschädigt. Herausgesprengte Teile des Knorpels infolge von Gewalteinwirkungen aufs Gelenk können sich auch im Gelenkspalt verklemmen und so zu starken Schmerzen und Bewegungseinschränkungen führen.

Abhilfe für eine betroffene Person kann hier durch eine Hüftgelenksspiegelung in Verbindung mit einer Knorpeltherapie geschaffen werden. Die Knorpeltherapie am Hüftgelenk gestaltet sich allerdings wegen der anatomischen Verhältnisse deutlich schwieriger, da eine Zugänglichkeit wie beispielsweise am Kniegelenk nicht so gegeben ist, da das Hüftgelenk im Gegensatz zum Kniegelenk ein Kugelgelenk ausbildet. Um eine ausreichende Zugänglichkeit des Hüftgelenks zu gewährleisten, erfordert es einen hohen technischen Aufwand, insbesondere für eine sichere Distraktion des Gelenks. Das Hüftgelenk ist im Wesentlichen in zwei Bereiche aufgeteilt. Der zentrale Bereich liegt zwischen Hüftkopf und Hüftpfanne. Da die Hüftpfanne den Hüftkopf stark umschließt und die Gelenklippe wie ein Saugnapf wirkt, muss bei der Hüftgelenksspiegelung der Hüftkopf über eine Zugvorrichtung (Distraktor) aus der Pfanne herausgezogen werden (Distraktion). Selbst dann ist der entstehende Raum sehr eng bemessen und der Zugang für ein medizinisches Instrument sehr schwierig. Um in den Raum zwischen den Hüftkopf und die Hüftpfanne zu gelangen, müssen daher teilweise gebogene beziehungsweise gekrümmte medizinische Instrumente beziehungsweise Werkzeuge verwendet werden.

Im Stand der Technik sind unterschiedliche medizinische Werkzeuge zur intrakorporalen Injektion eines Fluids bereits bekannt. Im Allgemeinen ist allen medizinischen Werkzeugen gemeinsam, dass diese eine Hohlkanüle mit wenigsten einem, in der Hohlkanüle anordenbaren, flexiblem Schlauchelement aufweisen. Hierbei weisen die Hohlkanüle und das Schlauchelement jeweils ein proximales - dem Körperzentrum im Wesentlichen zugewandtes - Ende und ein distales - dem Körperzentrum im Wesentlichen abgewandtes - Ende auf. Ferner ist zumindest auch ein Handgriff vorgesehen. Die Hohlkanüle umhüllt in der Regel das Schlauchelement auf einer bestimmten axialen Mantellänge vollständig, wobei das proximale Ende des Schlauchelements außerhalb der Mantellänge eine insbesondere vorgeformte Krümmung ausbilden kann. Die Hohlkanüle kann relativ zu dem Schlauchelement verschiebbar angeordnet sein. Zudem kann ein Verstellelement vorgesehen sein, wodurch die auf das Schlauchelement einwirkende beziehungsweise die das Schlauchelement wirksam umhüllende Mantellänge einstellbar ist. Die Ausbildung des medizinischen Werkzeugs beziehungsweise die Anordnung der Hohlkanüle relativ zu dem Schlauchelement beeinflusst damit die Art und Weise des benötigten Raums für die exakte Positionierung eines fluidischen Stoffes, insbesondere eines fluidischen Kollagens, innerhalb eines menschlichen Hüftgelenks.

Im Stand der Technik sind nun unterschiedliche medizinische Werkzeuge zur intrakorporalen Injektion eines Fluids bereits bekannt.

Aus der EP 1 054 703 B1, von der die Erfindung ausgeht, ist bereits ein medizinisches Werkzeug zur intrakorporalen Injektion eines Fluids bekannt, wobei durch ein axial verschiebbares Verstellelement die ein Schlauchelement wirksam umhüllende Mantellänge einer Hohlkanüle verändert werden kann. Ein Gehäuseteil/Handgriff weist hierbei zunächst eine stationäre Außenkomponente auf, die mit einem proximalen Ende der Hohlkanüle über eine Art Flansch verbunden ist. Das Verstellelement ist axial verschiebbar im Gehäuseteil/Handgriff gelagert. Das Verstellelement weist ferner ein "Daumenstück" auf, das der Operateur zum Vorschieben oder Zurückziehen der Hohlkanüle betätigen kann. Somit kann die Hohlkanüle relativ zum Schlauchelement bewegt und die wirksame Mantellänge der Hohlkanüle verändert werden und das innenliegende Schlauchelement kann so teilweise "freigelegt" werden. Hierdurch kann das Schlauchelement außerhalb der Mantellänge der Hohlkanüle eine vorgeformte Krümmung reversibel ausbilden.

Das zuvor beschriebene medizinische Werkzeug, von dem die Erfindung ausgeht, ist aber noch nicht optimal ausgebildet. Durch die hier realisierte Anordnung und Bedienung des Verstellelementes kann ein "Stick-Slip-Effekt" beziehungsweise ein Ruckgleiten hervorgerufen werden. Dies kann bei der Bewegung der Hohlkanüle in den äußerst geringen Raum innerhalb des Hüftgelenks zu einer unbeabsichtigten und unerwünschten Kollision führen. Aufgrund der Ausbildung dieses medizinischen Werkzeugs beziehungsweise aufgrund der Anordnung der Hohlkanüle mit den dortigen zusätzlichen Komponenten, insbesondere den stationären Außenkomponenten, wird das Werkzeug auch derart schwer und unhandlich, so dass es für den Operateur schwierig und ermüdend ist, das Werkzeug exakt zu führen. Insbesondere muss der Operateur das Werkzeug mit seinen beiden Händen betätigen beziehungsweise bedienen. Weiterhin wird auch durch das häufigere Ablegen beziehungsweise Aufnehmen des medizinischen Werkzeugs und der damit verbundenen Montage und Demontage von weiteren Hilfsmitteln ein erhöhter Zeitaufwand zur Durchführung des minimal-invasiven Eingriffs benötigt. Dies führt zum einen zu hohen Kosten und ist zum anderen auch eine zusätzliche Belastung für den Patienten. Das aus dem Stand der Technik bekannte medizinische Werkzeug zur intrakorporalen Injektion eines Fluids, von dem die Erfindung ausgeht, ist daher noch nicht optimal ausgebildet.

Der Erfindung liegt daher die Aufgabe zugrunde, ein mehrteiliges medizinisches Werkzeug zur intrakorporalen Injektion eines Fluids, insbesondere zum Positionieren eines flüssigen Kollagens in einem menschlichen Gelenk, insbesondere einem Hüftgelenk oder einem Schultergelenk nun derart auszugestalten und weiterzubilden, so dass einerseits das medizinische Werkzeug durch den Operateur mit einer Hand insbesondere über eine längere Zeitdauer ermüdungsfrei führbar und/oder betätigbar ist und ebenso andererseits eine exakte Positionierung des Fluids beziehungsweise des fluidischen Implantats innerhalb eines menschlichen Gelenks ermöglicht ist, insbesondere auch die Dauer des Eingriffs, die damit verbundenen Kosten beziehungsweise die für den Patienten mit dem Eingriff einhergehenden Belastungen verringert sind.

Die zuvor aufgezeigte Aufgabe ist nun zunächst für das eingangs genannte medizinische Werkzeug durch die Merkmale des Patentanspruchs 1 gelöst.

Die vorliegende Erfindung betrifft ein mehrteiliges medizinisches Werkzeug zur intrakorporalen Injektion eines Fluids, insbesondere zum Positionieren eines flüssigen und/oder zähflüssigen Kollagens in einem menschlichen Gelenk, insbesondere in einem Hüftgelenk, Kniegelenk oder in einem Schultergelenk, mit mindestens einer Hohlkanüle, mit mindestens einem in der Hohlkanüle anordenbaren flexiblen Schlauchelement, mit mindestens einem Gehäuseteil und mit mindestens einem Verstellelement, wobei die Hohlkanüle und das Schlauchelement jeweils ein proximales Ende und ein distales Ende aufweisen, wobei die Hohlkanüle - im angeordneten Zustand des Schlauchelements - das Schlauchelement auf einer Mantellänge zumindest teilweise umhüllt und das proximale Ende des Schlauchelements außerhalb der Mantellänge einen zumindest teilweise gekrümmten Verlauf ausbildet, wobei die Hohlkanüle relativ zu dem Schlauchelement verschiebbar und/oder bewegbar angeordnet ist, wobei das Verstellelement mit der Hohlkanüle funktional wirksam verbindbar und/oder verbunden ist, und wobei durch die Betätigung des Verstellelementes die Hohlkanüle relativ zum Schlauchelement bewegbar und/oder verschiebbar ist, die wirksame Mantellänge einstellbar ist, wobei das Verstellelement im Bereich des Gehäuseteils drehbar gelagert ist.

Das Grundprinzip der Erfindung geht zunächst dahin, dass das Verstellelement im Bereich des Gehäuseteils drehbar gelagert ist. Hierdurch können eine Vielzahl von Vorteilen realisiert werden, insbesondere kann der Operateur nun mit nur einer Hand das medizinische Werkzeug auf einfache Weise betätigen beziehungsweise bedienen, was im Folgenden noch näher erläutert werden darf.

Das Verstellelement ist auf oder an dem Gehäuseteil um die Rotationsachse der Hohlkanüle drehbar gelagert, insbesondere also grundsätzlich nicht - wie bisher im Stand der Technik - als axial gleitendes Element mit zusätzlichen weiteren Komponenten ausgeführt. Hierdurch wird das Auftreten eines "Stick-Slip-Effekts" nahezu vollständig vermieden. Es wird hierdurch nicht nur zusätzlich das Gewicht des medizinischen Werkzeugs optimiert, sondern auch die Gesamtlänge des medizinischen Werkzeugs kann entsprechend reduziert werden.

Dadurch, dass sich das Verstellelement axial an der proximalen Stirnseite des Gehäuseteils abstützt, kann es über ein vorgesehenes Gewinde einen axialen Vorschub oder Rückschub auf die Hohlkanüle erzeugen und somit kann dann die wirksame Mantellänge der Hohlkanüle entsprechend verändert werden, so dass dann das proximale Ende des Schlauchelementes in einer gekrümmten Form "freiliegt" beziehungsweise die Krümmung und/oder Länge des freiliegenden proximalen Endes des Schlauchelementes mit Hilfe des Verstellelementes einstellbar und/oder steuerbar ist.

Mit dem Begriff "wirksame Mantellänge (M)" der Hohlkanüle ist im Wesentlichen der Abschnitt der Hohlkanüle gemeint, der sich außerhalb des Gehäuseteils und/oder außerhalb des Verstellelementes in Richtung des proximalen Endes des Schlauchelementes erstreckt und insbesondere auf diesem Abschnitt das Schlauchelement entsprechend umhüllt. Insbesondere diese wirksame Mantellänge (M) ist entsprechend durch die mit Hilfe des Verstellelementes einstellbare jeweilige Längsverschiebung der Hohlkanüle vom Operateur einstellbar, insbesondere so dass das proximale Ende des Schlauchelementes in einer gekrümmten Form freilegbar ist, was im Folgenden nochmals näher beschrieben wird.

Weiterhin kann in bevorzugter Ausführungsform insbesondere durch das Gewinde eine präzisere Einstellung der wirksamen Mantellänge der Hohlkanüle und somit - hierdurch resultierend - auch des gekrümmten Verlaufes des freiliegenden proximalen Endes des Schlauchelements außerhalb der Mantellänge der Hohlkanüle eingestellt werden.

Das Gehäuseteil weist bevorzugt eine Aufnahme auf und das distale Ende des Schlauchelementes ist hierbei in der Aufnahme anordenbar beziehungsweise in der Aufnahme angeordnet, insbesondere dort drehfest anordenbar.

Das flexibel ausgebildete Schlauchelement weist in bevorzugter Ausführungsform insbesondere ein Formgedächtnismaterial auf beziehungsweise ist aus einem Formgedächtnismaterial hergestellt oder ist aus einem elastisch verformbaren Material hergestellt, wobei das Schlauchelement mindestens eine vorgeformte Krümmung aufweist. Das Schlauchelement weist bevorzugt eine vorgeformte Krümmung beziehungsweise Biegung auf und wird insbesondere im Bereich der im Wesentlichen gradlinig ausgebildeten Hohlkanüle auch in die entsprechende im Wesentlichen gradlinige Form, insbesondere entlang der einstellbaren wirksamen Mantellänge der Hohlkanüle "gezwungen". Sobald das Schlauchelement die Hohlkanüle verlässt beziehungsweise die Hohlkanüle relativ zum Schlauchelement entsprechend bewegt wird, insbesondere die wirksame Mantellänge verringert wird, krümmt sich der entsprechende freiliegende Bereich des Schlauchelementes dann zur vorgeformten Biegung beziehungsweise Krümmung, insbesondere krümmt sich daher das proximale Ende des Schlauchelementes, wenn dieses von der Hohlkanüle "freigelegt" worden ist.

Zur weiteren Realisierung der axialen Bewegung der Hohlkanüle ist in bevorzugter Ausführungsform von deren distalen Ende ausgehend zumindest eine sich axial über einen Teil der Hohlkanüle erstreckende Nut vorgesehen.

Das Gehäuseteil weist bevorzugt eine Kavität auf, wobei die Kavität insbesondere als Keilnabenbuchse ausgeführt ist oder in die Kavität eine Keilnabenbuchse eingesetzt ist. Das Gehäuseteil greift bevorzugt daher funktional wirksam zumindest teilweise in die Nut, insbesondere mit mindestens einem Fortsatz, ein und verhindert somit eine Rotation der Hohlkanüle während einer durch das Verstellelement aufgezwungenen Bewegung der Hohlkanüle beziehungsweise wird dadurch die axiale Bewegung der Hohlkanüle realisiert.

Ferner weist das distale Ende des Schlauchelements in bevorzugter Ausführungsform insbesondere ein Befestigungselement auf, welches vorzugsweise als eine Rändelmutter, besonders bevorzugt als eine Rändelhohlmutter ausgeführt ist. Mittels des Befestigungselements, insbesondere der Rändelmutter oder der Rändelhohlmutter, kann das Schlauchelement insbesondere verdrehsicher in der Aufnahme am beziehungsweise im Gehäuseteil eingesetzt und/oder dort fixiert werden. Somit ist insbesondere auch eine relative Winkellage des Schlauchelements zur Hohlkanüle mittels des Befestigungselements einstellbar. Das Befestigungselement kann insbesondere auch als integraler Bestandteil des Schlauchelementes ausgebildet sein.

Durch das derart ausgebildete mehrteilige medizinische Werkzeug ist dieses einerseits durch den Operateur mit einer Hand über eine längere Zeitdauer ermüdungsfrei führbar und/oder betätigbar und andererseits ist auch eine exakte Positionierung des Fluids, insbesondere des fluidischen beziehungsweise flüssigen Knorpelimplantats, innerhalb eines menschlichen Gelenks, insbesondere eines Hüftgelenks, ermöglicht. Ferner wird die Dauer des Eingriffs und es werden die hierdurch verbundenen Kosten verringert, insbesondere die Belastung des Patienten ist verringert. Weiterhin ist, insbesondere und bevorzugt durch die zwischen dem Verstellelement und der Hohlkanüle wirksam vorgesehene Gewindeverbindung, eine präzisere Einstellung der wirksamen Mantellänge der Hohlkanüle realisierbar und somit resultierend auch der gekrümmte Verlauf des proximalen Endes des inneren Schlauchelements außerhalb der Hohlkanüle entsprechend einstellbar beziehungsweise das "freie Ende" (Länge und/oder Krümmung) des Schlauchelementes sehr genau steuerbar und/oder einstellbar.

Im Ergebnis sind die eingangs genannten Nachteile vermieden und entsprechende Vorteile erzielt.

Es gibt nun eine Vielzahl von Möglichkeiten, das erfindungsgemäße medizinische Werkzeug in vorteilhafter Art und Weise auszugestalten und weiterzubilden. Hierzu darf zunächst auf die dem Patentanspruch 1 nachgeordneten Patentansprüche verwiesen werden.

Im Folgenden darf eine bevorzugte Ausführungsform der Erfindung anhand der Zeichnungen und der dazugehörigen Beschreibung näher erläutert werden. In den Zeichnungen zeigen:
- Fig. 1: eine vereinfachte schematische Darstellung, teilweise eine Schnittdarstellung, eines erfindungsgemäßen mehrteiligen medizinischen Werkzeugs mit der Darstellung der wesentlichen Komponenten im unmontierten Zustand, aber mit Darstellung der einstellbaren beziehungsweise wirksamen Mantellänge M,
- Fig. 2: eine vereinfachte schematische Darstellung des Querschnitts beziehungsweise der Schnittebene entlang der Linie A-A in Fig. 1,
- Fig. 3a, 3b: eine vereinfachte schematische Darstellung des Querschnitts beziehungsweise der Schnittebene entlang der Linie B-B in Fig. 1 für eine erste (vergleiche Fig. 3a) und eine zweite (vergleiche Fig. 3b) Ausführungsform eines Verstellelementes,
- Fig. 4: eine vereinfachte schematische Darstellung des Querschnitts beziehungsweise der Schnittebene entlang der Linie C-C in Fig. 1,
- Fig. 5: in schematischer Darstellung, teilweise als eine Schnittdarstellung die bevorzugte Ausführungsform des mehrteiligen medizinischen Werkzeuges aus Fig. 1 im Zusammenbau beziehungsweise im Gebrauchszustand mit vollständig ausgefahrener Hohlkanüle (mit maximaler wirksamer Mantellänge M),
- Fig. 6: in schematischer Darstellung, teilweise als eine Schnittdarstellung die bevorzugte Ausführungsform des mehrteiligen medizinischen Werkzeuges aus Fig. 1 im Zusammenbau beziehungsweise im Gebrauchszustand, mit vollständig eingefahrener Hohlkanüle (mit minimaler wirksamer Mantellänge M), und
- Fig.7: in schematischer Darstellung die bevorzugte Ausführungsform des mehrteiligen medizinischen Werkzeuges aus Fig. 6 im Gebrauchszustand während der Applikation an einem Hüftgelenk.

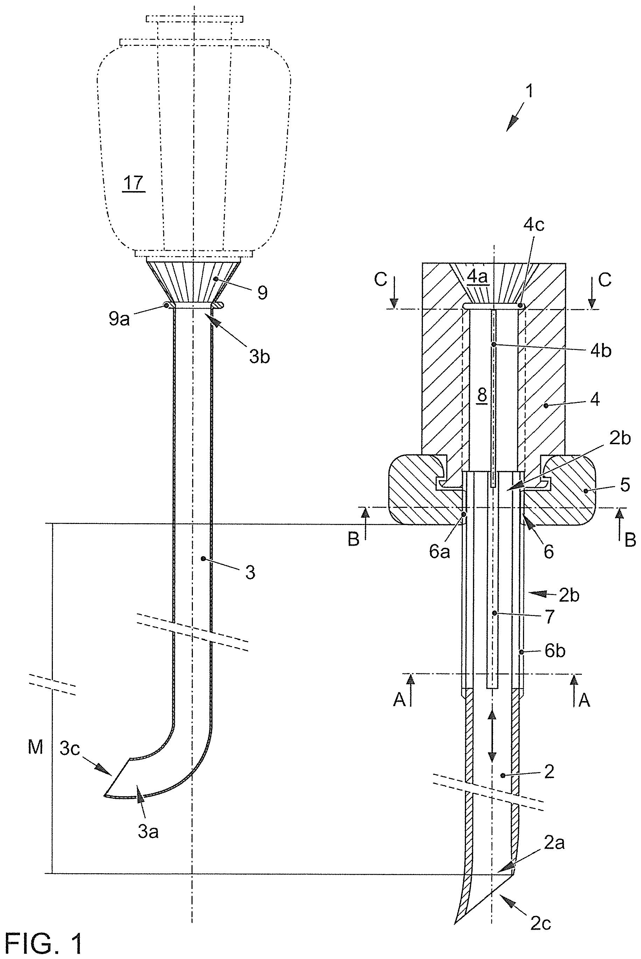

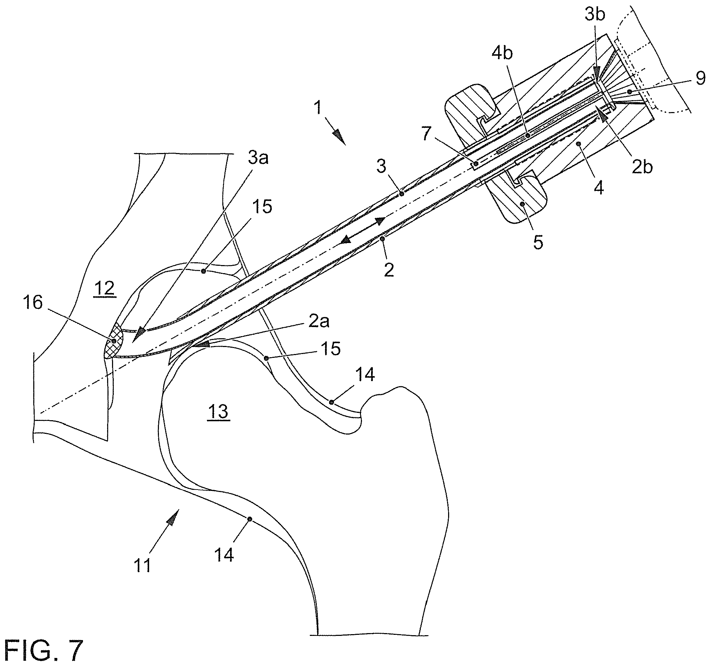

Die Fig. 1 bis 7 zeigen zunächst in jeweiliger vereinfachter schematischer Darstellung beziehungsweise in jeweiliger Schnittdarstellung - zumindest teilweise - ein mehrteiliges medizinisches Werkzeug 1 zur intrakorporalen Injektion eines Fluids, insbesondere zum Positionieren eines flüssigen und/oder zähflüssigen Kollagens in einem menschlichen Gelenk, insbesondere hier in einem Hüftgelenk 11 (vergleiche Fig. 7) oder in einem Schultergelenk.

Denkbar ist auch, dass das hier mehrteilig aufgebaute medizinische Werkzeug 1 auch für andere Gelenke, beispielsweise bei einem Kniegelenk zum Einsatz kommt.

Das medizinische Werkzeug 1 ist insbesondere mehrteilig aufgebaut und weist - wie aus der Fig. 1 ersichtlich - eine Hohlkanüle 2 mit einem proximalen Ende 2a und mit einem distalen Ende 2b auf. Das proximale Ende 2a weist vorzugsweise eine leichte, hier nicht näher bezeichnete Krümmung und insbesondere eine angeschrägte Öffnung 2c auf.

Ferner ist ein flexibles Schlauchelement 3 mit einem proximalen Ende 3a und mit einem distalen Ende 3b erkennbar beziehungsweise in den Fig. 1, 5, 6 und 7 dargestellt. Vorzugsweise ist das proximale Ende 3a auch mit einer angeschrägten Öffnung 3c versehen. Das flexible Schlauchelement 3 ist im Durchmesser etwas geringer bemessen als die Hohlkanüle 2, damit das Schlauchelement 3 sich einfach in die Hohlkanüle 2 einführen lässt sowie auch insbesondere eine möglichst widerstandsfreie Relativbewegung zwischen der Hohlkanüle 2 und dem Schlauchelement 3 durchführbar ist. Weiterhin ist ein Gehäuseteil 4 vorgesehen beziehungsweise vorhanden. Das "Gehäuseteil 4" ist auch als "Gehäuse" bezeichenbar.

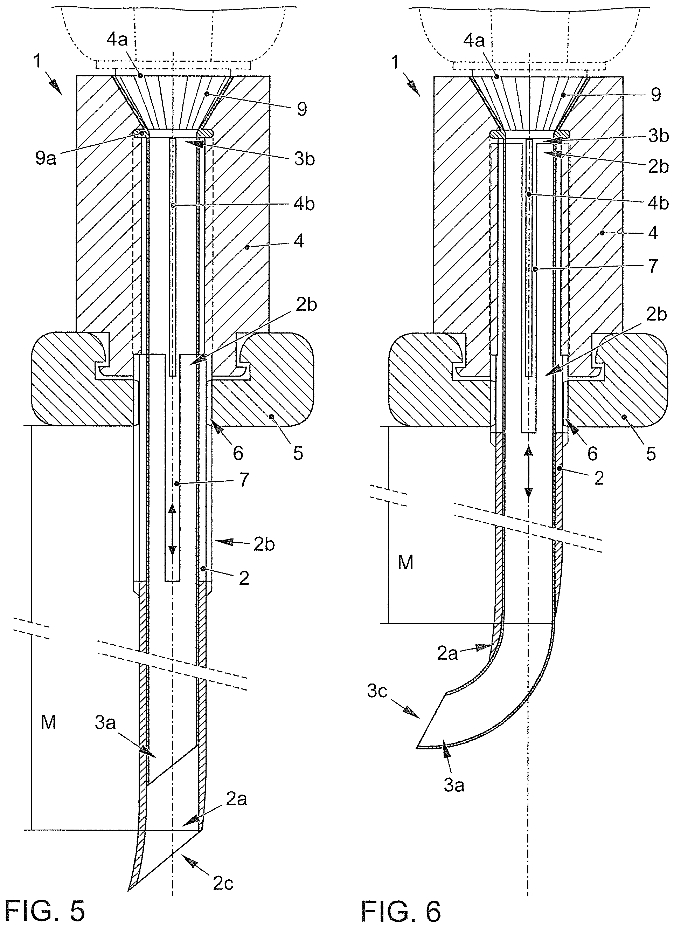

Die Hohlkanüle 2 ist vorzugsweise im Wesentlichen gerade und starr, aber insbesondere auch mit einer Krümmung an dem proximalen Ende 2a, ausgebildet und umhüllt im Zusammenbau beziehungsweise im Gebrauchszustand des medizinischen Werkzeugs 1 das innere Schlauchelement 3 zumindest teilweise (vergleiche Fig. 5 und 6), wobei die jeweilige insbesondere in den Fig. 5 und 6 dargestellte Mantellänge M einstellbar beziehungsweise veränderbar ist.

Das proximale Ende 3a des inneren Schlauchelements 3 bildet außerhalb der Mantellänge M einen gekrümmten Verlauf aus beziehungsweise nimmt wieder seine vorgeformte Krümmung ein. Hierzu weist das Schlauchelement 3 ein Formgedächtnismaterial auf beziehungsweise ist aus einem Formgedächtnismaterial oder einem elastischen Material hergestellt, vorzugsweise aus einem Kunststoffmaterial beziehungsweise einem Polymer, wobei das Schlauchelement 3 beziehungsweise das Material mindestens eine vorgeformte Krümmung/Biegung aufweist. Anders ausgedrückt, im nicht durch die Hohlkanüle 2 umhüllten Zustand des Schlauchelementes 3, nimmt dann das Schlauchelement 3 beziehungsweise der freiliegende proximale Bereich des Schlauchelementes 3 eine vorgeformte beziehungsweise eine vorgebogene Krümmung und/oder einen gekrümmten Verlauf ein.

Die eingangs genannten Nachteile sind nun zunächst dadurch vermieden, das Verstellelement 5 im Bereich des Gehäuseteils 4 drehbar gelagert ist. Hierdurch werden die eingangs genannten Nachteile vermieden und die entsprechenden bereits erwähnten Vorteile realisiert.

Das Gehäuseteil 4 weist eine Aufnahme 4a auf, wobei das distale Ende 3b des Schlauchelementes 3 in der Aufnahme 4a anordenbar ist beziehungsweise in der Aufnahme 4a angeordnet ist, insbesondere hier verdrehsicher anordenbar ist. Das Gehäuseteil 4 ist insbesondere hülsenförmig ausgebildet.

Insbesondere ist aus den Fig. 1, 5 und 6 ersichtlich, dass im oberen Bereich des Gehäuseteils 4 die Aufnahme 4a für das distale Ende 3b des Schlauchelementes 3 vorgesehen ist. Hierbei ist mit dem "oberen Bereich" des Gehäuseteils 4 der eher "distale Bereich" des Gehäuseteils 4 gemeint.

Es ist ferner ein Verstellelement 5 erkennbar, wobei das Verstellelement 5 auf dem Gehäuseteil 4, insbesondere um die Rotationsachse der Hohlkanüle 2 drehbar gelagert ist. Zu erkennen ist auch, dass sich das Verstellelement 5 axial an der proximalen Stirnseite des Gehäuseteils 4 abstützt. Insbesondere ist das Verstellelement 5 an der proximalen Seite des Gehäuseteils 4 drehbar angeordnet.

Das Verstellelement 5 und das distale Ende 2b (beziehungsweise der distale Endbereich) der äußeren Hohlkanüle 2 ist über ein hier in den Fig. 1 bis 7 nur teilweise schematisch angedeutetes Gewinde/eine Gewindeverbindung 6 in funktionaler Wirkverbindung, wobei das Verstellelement 5 hierfür ein Innengewinde 6a und das distale Ende 2b (beziehungsweise der distale Endbereich) der Hohlkanüle 2 hierfür ein Außengewinde 6b aufweist und das Innengewinde 6a mit dem Außengewinde 6b in Eingriff steht. Hierbei sind die jeweiligen Innen- und Außengewinde 6a, 6b (beziehungsweise die so gebildete "Gewindeverbindung 6") vorzugsweise als Feingewinde (beziehungsweise als eine Feingewindeverbindung) ausgebildet.

Die Fig. 1, 5 und 6 zeigen, dass von dem distalen Ende 2b der Hohlkanüle 2 ausgehend, mindestens eine sich axial über zumindest einen Teil der Hohlkanüle 2 erstreckende Nut 7 vorhanden ist. Insbesondere sind über den Umfang verteilt hier insgesamt vier Nuten 7 vorgesehen. Die "Nuten 7" können auch als "Führungsnuten" bezeichnet werden, wobei die Nuten 7 insbesondere als Ausnehmungen beziehungsweise Schlitze in der Hohlkanüle 2 ausgebildet sind.

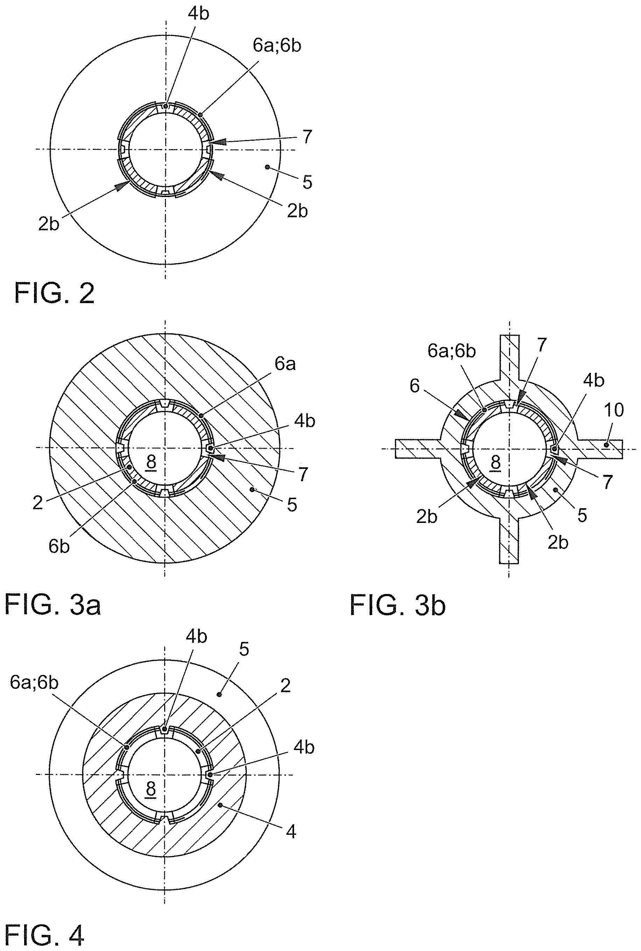

Die Fig. 1, 5, 6 und 7 zeigen weiter, dass das Gehäuseteil 4 eine sich im Wesentlichen über die gesamte Länge des Gehäuseteils 4 erstreckende Kavität 8 aufweist. Vorzugsweise ist die Kavität 8 zur Aufnahme beziehungsweise zur Positionierung des distalen Endes 2b der Hohlkanüle 2 vorgesehen. Die Kavität 8 ist hierzu insbesondere als eine Art Keilnabenbuchse mit einem entsprechenden Keilnabenprofil ausgeführt (vergleiche auch Fig. 4), wobei das Profil der Keilnabenbuchse beziehungsweise hier mindestens ein Fortsatz 4b, insbesondere schematisch angedeutete keilartige Fortsätze 4b, in die zumindest eine Nut 7 beziehungsweise in die Nuten 7 der Hohlkanüle 2 funktional wirksam eingreift beziehungsweise eingreifen.

Die Fig. 5 und 6 zeigen, dass das distale Ende 2b beziehungsweise der distale Endbereich der Hohlkanüle 2 in der Kavität 8 des Gehäuseteils 4 positioniert ist. Insbesondere das distale Ende 2b (beziehungsweise der distale Bereich) ist teilweise in der Kavität 8 (vergleiche Fig. 5) beziehungsweise nahezu vollständig in der Kavität 8 (vergleiche Fig. 6) positioniert.

Ferner ist an dem distalen Ende 3b des Schlauchelements 3 ein Befestigungselement 9 vorgesehen und/oder ausgebildet, wobei das Befestigungselement 9 vorzugsweise als eine Art Rändelmutter, besonders bevorzugt als eine Rändelhohlmutter ausgeführt ist. Das distale Ende 3b des Schlauchelements 3 ist mittels der Rändelmutter 9 oder der Rändelhohlmutter in der Aufnahme 4a des Gehäuseteils 4 insbesondere verdrehsicher einsetzbar und hierdurch ist dann auch die Winkellage des inneren Schlauchelements 3 relativ zur Hohlkanüle 2 einstellbar, insbesondere auch fixierbar. Hierzu weist die Aufnahme 4a insbesondere eine Innenkontur, insbesondere eine Innen-Oberflächenkontur korrespondierend zu der Außenkontur beziehungsweise der Außen-Oberflächenkontur des Befestigungselement 9 beziehungsweise insbesondere zu der Rändel-(hohl)mutter auf.

Insbesondere aus der Fig. 1 ist auch gut erkennbar, dass der distale Bereich des Schlauchelementes 3 beziehungsweise das Befestigungselement 9 einen wulstartigen Randbereich 9a aufweist, der eine entsprechende Flexibilität aufweist und innerhalb einer umlaufenden Ausnehmung 4c der Aufnahme 4a positionierbar ist. Hierdurch kann der distale Bereich des Schlauchelementes 3 insbesondere der wulstartige Randbereich 9a in die Ausnehmung 4c einfach eingeklipst und fixiert werden. Aufgrund der Einklipsung/Fixierung ist insbesondere auch eine Befestigung des Schlauchelementes 3 in axialer Richtung gewährleistet, wobei das Schlauchelement 3 unterschiedlichen Winkellagen aufgrund der profilierten Außen-Oberfläche des Befestigungselementes 9 und der profilierten InnenOberfläche der Aufnahme 4a verdrehsicher positioniert werden kann, da die Profile der Oberflächen entsprechend korrespondierend zueinander ausgebildet sind, so dass verschiedene Winkellagen des Schlauchelementes 3 realisierbar sind.

Die Fig. 2, 3a, 3b und 4 zeigen nun eine schematische, insbesondere auch jeweilige geschnittene Darstellungen eines jeweiligen Querschnitts in einer jeweiligen Schnittebene A, B und C entlang der in der Fig. 1 jeweiligen erkennbaren Linien.

Die Fig. 2 zeigt in der Schnittebene A das distale Ende 2b beziehungsweise den distalen Endbereich der Hohlkanüle 2, wobei ein Außengewinde 6b mit vier Nuten 7 als Aufnahme für das Keilnabenprofil des Gehäuseteils 4 vorgesehen beziehungsweise ausgebildet sind.

Die Fig. 3a und 3b zeigen in der Schnittebene B das distale Ende 2b der Hohlkanüle 2 und das Verstellelement 5 in einer ersten (vergleiche Fig. 3a), sowie einer zweiten Ausführungsform (vergleiche Fig. 3b). Wie bereits aus der Fig. 2 ersichtlich, ist hier im Zentrum des Verstellelementes 5 die Gewindeverbindung 6, insbesondere das Innengewinde 6a des Verstellelementes 5 und das Außengewinde 6b der Hohlkanüle 2 nur schematisch angedeutet, sowie aber auch die vier Nuten 7 und/oder die Fortsätze 4b erkennbar sind.

Aus Fig. 3a in Verbindung mit Fig. 1 ist nun ersichtlich, dass das Verstellelement 5 im Wesentlichen scheibenförmig ausgeführt ist. Zur weiteren Gewichtsreduktion des medizinischen Werkzeugs 1 ist auch eine alternative Ausgestaltung möglich, wie aus der Fig. 3b ersichtlich, insbesondere nämlich als eine Art Flügelrad, hier mit vier Flügeln 10.

Die Fig. 4 zeigt nun das Gehäuseteil 4 in der Schnittebene C, wobei hier gut das Keilnabenprofil mit vier keilartigen Fortsätzen 4b in der Kavität 8 des Gehäuseteils 4 zu erkennen ist. Denkbar ist allerdings auch eine Ausführung beispielsweise mit nur zwei gegenüberliegenden keilförmigen Fortsätzen oder nur einem Fortsatz zum Eingriff in eine jeweilige Nut.

Die Fig. 5 und Fig. 6 zeigen nun das mehrteilige medizinische Werkzeug 1 aus der Fig. 1 im Zusammenbau beziehungsweise im Gebrauchszustand. Das Schlauchelement 3 ist im Wesentlichen innerhalb der Hohlkanüle 2 angeordnet.

In Fig. 5 ist die Hohlkanüle 2 im Wesentlichen vollständig ausgefahren beziehungsweise aus dem Gehäuseteil 4 mit Hilfe des Verstellelementes 5 herausgedreht und umhüllt das innenliegende flexible Schlauchelement 3 mit der maximalen wirksamen Mantellänge M in der Längsrichtung mit Ausnahme des Bereiches des Schlauchelementes 3, der sich innerhalb des Gehäuseteils 4 erstreckt, im Wesentlichen vollständig. Unter der wirksamen Mantellänge M wird also insbesondere der Bereich der Hohlkanüle 2 verstanden, der sich aus dem Gehäuseteil 4 in Längsrichtung (wie in den Fig. 5 und 6 dargestellt nach unten aus dem Gehäuseteil 4) heraus erstreckt.

Die Fig. 6 hingegen zeigt die Hohlkanüle 2 im Wesentlichen vollständig eingefahren beziehungsweise eingedreht mit der minimalen wirksamen Mantellänge M. Das innenliegende flexible Schlauchelement 3 wird hier nun nur teilweise in seiner Längsrichtung umhüllt, denn das proximale Ende 3a beziehungsweise der proximale Endbereich des Schlauchelementes 3 liegt hier "frei" und kann eine bereits vorgegebene beziehungsweise in das Material eingeprägte Krümmung reversibel einnehmen beziehungsweise bildet nun hier einen gekrümmten Verlauf, so wie in Fig. 6 dargestellt.

Je nachdem wie die wirksame Mantellänge M der Hohlkanüle 2 vom Operateur ausgewählt beziehungsweise eingestellt wird, bildet sich aufgrund der Zwangsführung durch die außenliegende Hohlkanüle 2 ein bestimmter gekrümmter Verlauf des freiliegenden - proximalen - Bereiches des Schlauchelementes 3 aus und die Positionierung der Öffnung 3c des proximale Endes 3a des flexiblen Schlauchelements 3 im Gelenk, insbesondere im Hüftgelenk 11, ändert sich beziehungsweise kann vom Operateur entsprechend gesteuert werden.

Die Fig. 7 zeigt nun das mehrteilige medizinische Werkzeug 1 aus der Fig. 6 im Gebrauchszustand während der Applikation beziehungsweise der Injektion eines Fluids 16, insbesondere eines Kollagens in ein menschliches Hüftgelenk 11. Zur Vorbereitung für diesen minimal-invasiven Eingriff am Hüftgelenk 11, muss der Raum zwischen der Hüftgelenkpfanne 12 und dem Hüftgelenkkopf 13 zuerst mittels einer Distraktion erweitert werden.

Erkennbar ist in Fig. 7, dass das proximale Ende 2a der Hohlkanüle 2, die Gelenkkapsel 14 durchdrungen hat und zwischen der Hüftgelenkpfanne 12 und dem Hüftgelenkkopf 13 positioniert ist. Das proximale Ende 3a des flexiblen Schlauchelements 3 ist ebenfalls zwischen der Hüftgelenkpfanne 12 und dem Hüftgelenkkopf 13 positioniert, wobei das proximale Ende 3a beziehungsweise der proximale Bereich einen gekrümmten Verlauf aufzeigt beziehungsweise einnimmt. Durch die Vorgabe der wirksamen Mantellänge M ist der gekrümmte Verlauf und somit auch die Position beziehungsweise die Positionierung der Öffnung 3c des Schlauchelementes 3 durch den Operateur einstellbar beziehungsweise entsprechend steuerbar. Somit ist, wie hier dargestellt, ein im Bereich der Hüftgelenkpfanne 12 gelegener defekter Gelenkknorpel 15 erreichbar und das Fluid 16, insbesondere ein Kollagen an dieser Position auch injizierbar. Vorzugsweise kommt hier ein biologischer Stoff aus einem hochreinen Kollagen zum Einsatz. Der Knorpelschaden wird dadurch - im Endeffekt - vollständig geschlossen und das Gelenk wieder belastbar beziehungsweise bildet sich neuer Knorpel aus.

Aus den Fig. 1 bis 7 ist die Funktionsweise des mehrteiligen medizinischen Werkzeugs 1, das auch als "medizinisches Instrument" bezeichenbar ist, ersichtlich beziehungsweise anhand dieser zuvor genannten Figuren auch entsprechend beschrieben.

Insbesondere zeigt die Fig. 7, dass am distalen Ende 3b des Schlauchelementes 3 ein Fluid 16, insbesondere ein Kollagen in ein Gelenk, hier in ein Hüftgelenk 11 eingebracht werden kann. Mit Hilfe des medizinischen Werkzeuges 1 ist über das Schlauchelement 3, nämlich über die entsprechende Positionierung des proximalen Endes 3a beziehungsweise der Öffnung 3c des Schlauchelementes 3 an eine bestimmte Stelle, insbesondere an eine defekte Knorpelstelle ein flüssiges Kollagen injizierbar. Über die relative Verschiebung/Bewegung der Hohlkanüle 2 relativ zum Schlauchelement 3, insbesondere durch die rotatorische Betätigung des Verstellelementes 5, kann das proximale Ende 3a und/oder die Öffnung 3c des Schlauchelementes 3 entsprechend positioniert/gesteuert werden. Insbesondere kann das Schlauchelement 3 aber auch innerhalb der Hohlkanüle 2 verdreht werden, nämlich wenn insbesondere das Befestigungselement 9 in Fig. 7 nach rechts aus dem Gehäuseteil 4 herausgezogen wird, dann in eine bestimmte Position gedreht und wieder in die Aufnahme 4a des Gehäuseteils 4 zurück gedrückt wird. Insbesondere ist hier eine formschlüssige Aufnahme des Befestigungselementes 9 in der Aufnahme 4a, insbesondere in einer bestimmten gewünschten Winkelposition beziehungsweise in einer bestimmten Winkelposition von mehreren möglichen Winkelpositionen realisierbar. Über das drehbar angeordnete Verstellelement 5 ist eine einfache Handhabung des medizinischen Werkzeuges 1 ermöglicht, insbesondere eine Bewegung der Hohlkanüle 2 in Längsrichtung und damit eine relative Bewegung der Hohlkanüle 2 zum Schlauchelement 3 ermöglicht, wie zuvor beschrieben beziehungsweise aus den Fig. 1 bis 7 und den dortigen Pfeilen ersichtlich dargestellt. Vom besonderen Vorteil ist, wenn das Schlauchelement 3 eine vorgeformte Biegung/Krümmung aufweist, insbesondere auch eine vorgeformte Spiralform aufweist. Damit kann das Schlauchelement 3 in einer bestimmten Länge vom Operateur abgeschnitten und in die Hohlkanüle 2 eingesetzt werden beziehungsweise dann bei der "Freigabe" des Schlauchelementes 3 aus der Hohlkanüle 2 auch eine vom Operateur gewünschte spezifische Krümmung realisiert werden.

Ganz grundsätzlich darf an dieser Stelle nochmals zu dem in den Fig. 1 bis 7 dargestellten mehrteiligen Werkzeug 1 ausgeführt beziehungsweise darauf hingewiesen werden, dass hier das Gehäuseteil 4 nur schematisch dargestellt ist und es eine Vielzahl von möglichen Ausführungsformen für das Gehäuseteil 4 geben kann. Insbesondere kann das Gehäuseteil 4 beziehungsweise das hier als "Gehäuseteil 4" bezeichnete "Bauteil 4" hülsenförmig ausgebildet sein und/oder für das Zusammenwirken mit einem Finger und/oder einem Daumen und/oder einer menschlichen Hand entsprechende weitere Stege und/oder Griffe aufweisen, insbesondere teilweise auch als ein "Handgriff" ausgebildet sein. Auch kann das hier in den Fig. 1 bis 7 dargestellte Gehäuseteil 4 als Teilkomponente und/oder Zusatzkomponente eines "vergrößerten Handgriffes" ausgebildet sein. Unter der Bezeichnung "Gehäuseteil 4" sind damit im Wesentlichen, insbesondere auch alle Bauteile umfasst beziehungsweise gemeint, die vom Operateur manuell ergriffen werden können, wobei insbesondere dann aber das Verstellelement 5 manuell betätigbar ist.

Insbesondere kann beziehungsweise wird bei dem in den Fig. 1 bis 7 dargestellten Werkzeug 1 dann das distale Ende 3b des Schlauchelementes 3 mit einer Spritzeinheit fluidtechnisch wirksam strömungsverbunden beziehungsweise ist - bei Einsatz des Werkzeuges 1 - mit einer hier nicht dargestellten Spritzeinheit fluidtechnisch strömungsverbunden, so dass das Fluid, insbesondere das flüssige Kollagen in das distale Ende 3b des Schlauchelementes 3 einbringbar ist und bis zum proximalen Ende 3a des Schlauchelementes 3 förderbar ist. Für die Anordnung einer Spritzeinheit und/oder für die strömungstechnische Verbindung der Spritzeinheit mit dem Werkzeug 1 sind unterschiedliche Möglichkeiten denkbar. Insbesondere kann das Gehäuseteil 4 hierbei, als ein Teil-Handgriff ausgebildet sein und/oder in die Abgabeöffnung einer entsprechenden Spritzeinheit, insbesondere in eine Spritze eingesteckt werden, die vom Operateur entsprechend manuell bedienbar und/oder ergreifbar ist. Zwischen dem Gehäuseteil 4 und einer Spritzeinheit können dann entsprechende Dichtelemente vorgesehen, die hier nicht dargestellt sind.

Insbesondere ist aber, was durch die gestrichelte Darstellung in Fig. 1 (links) angedeutet sein soll, am distalen Ende 3b beziehungsweise am distalen Bereich des Schlauchelementes 3 ein Adapter und/oder ein Adapteranschluss 17 vorgesehen und/oder ausgebildet, der mit einer Spritzeinheit, insbesondere mit einer Spritze strömungstechnisch verbindbar ist. Ein derartiger Adapter/Adapteranschluss 17 ist aus Gründen der Übersichtlichkeit aber nur in Fig. 1 (links) beziehungsweise Fig. 7 rechts gestrichelt dargestellt. Dieser Adapter 17 kann als eine bekannte "Luer-Lock"-Verbindung ausgebildet sein beziehungsweise einen derartigen Luer-Lock-Anschluss aufweisen. Wobei der Adapter 17 dann insbesondere als ein integraler Bestandteil des Schlauchelementes 3 ausgebildet ist beziehungsweise sein kann.

Im Endeffekt sind die eingangs genannten Nachteile vermieden und es sind entsprechende Vorteile erzielt, insbesondere ist einerseits das medizinische Werkzeug durch den behandelnden Arzt mit einer Hand über eine längere Zeitdauer ermüdungsfrei führbar und ebenso eine exakte Positionierung des fluidischen Stoffes, insbesondere eines Kollagens innerhalb eines menschlichen Hüftgelenks möglich. Ferner wird die Dauer des Eingriffs und die damit verbundenen Kosten, insbesondere aber die Belastung des Patienten verringert.

### BEZUGSZEICHENLISTE

- 1: Mehrteiliges medizinisches Werkzeug
- 2: äußere Hohlkanüle
- 2a: proximales Ende der äußeren Hohlkanüle
- 2b: distales Ende der äußeren Hohlkanüle
- 2c: Öffnung
- 3: inneres flexibles Schlauchelement
- 3a: proximales Ende des inneren Schlauchelements
- 3b: distales Ende des inneren Schlauchelements
- 3c: Öffnung
- 4: Gehäuseteil
- 4a: Aufnahme
- 4b: Fortsatz
- 4c: Ausnehmung
- 5: Verstellelement
- 6: Gewinde/Gewindeverbindung
- 6a: Innengewinde
- 6b: Außengewinde
- 7: Nut
- 8: Kavität im Gehäuseteil
- 9: Befestigungselement
- 9a: wulstartiger Randbereich
- 10: Flügel
- 11: Hüftgelenk
- 12: Hüftgelenkpfanne
- 13: Hüftgelenkkopf
- 14: Gelenkkapsel
- 15: Gelenkknorpel
- 16: Fluid, flüssiger Stoff, insbesondere Kollagen
- 17: Adapter/Adapteranschluss
- M: Mantellänge

## Patentansprüche

1. Mehrteiliges medizinisches Werkzeug (1) zur intrakorporalen Injektion eines Fluids (16), insbesondere zum Positionieren eines flüssigen und/oder zähflüssigen Kollagens in einem menschlichen Gelenk, insbesondere in einem Hüftgelenk (11) oder in einem Schultergelenk, mit mindestens einer Hohlkanüle (2), mit mindestens einem in der Hohlkanüle (2) anordenbaren flexiblem Schlauchelement (3), mit mindestens einem Gehäuseteil (4) und mit mindestens einem Verstellelement (5), wobei die Hohlkanüle (2) und das Schlauchelement (3) jeweils ein proximales Ende (2a, 3a) und ein distales Ende (2b, 3b) aufweisen, wobei die Hohlkanüle (2) - im angeordneten Zustand des Schlauchelements (3) - das Schlauchelement (3) auf einer Mantellänge (M) zumindest teilweise umhüllt und das proximale Ende (3a) des Schlauchelements (3) außerhalb der Mantellänge (M) einen zumindest teilweise gekrümmten Verlauf ausbildet, wobei die Hohlkanüle (2) relativ zu dem Schlauchelement (3) verschiebbar und/oder bewegbar angeordnet ist, wobei das Verstellelement (5) mit der Hohlkanüle (2) funktional wirksam verbindbar und/oder verbunden ist, und wobei durch die Betätigung des Verstellelementes (5) die Hohlkanüle (2) relativ zum Schlauchelement (3) bewegbar und/oder verschiebbar ist und die wirksame Mantellänge (M) einstellbar ist, **dadurch gekennzeichnet, dass** das Verstellelement (5) im Bereich des Gehäuseteils (4) um die Rotationsachse der Hohlkanüle (2) drehbar gelagert ist und sich axial an der proximalen Stirnseite des Gehäuseteils (4) abstützt, sodass durch ein Drehen des Verstellelements (5) über ein Gewinde ein axialer Vorschub oder Rückschub der Hohlkanüle (2) erzeugt werden kann, um die wirksame Mantellänge (M) der Hohlkanüle (2) zu verändern und die Krümmung und/oder Länge des freiliegenden proximalen Endes (2a, 3a) des Schlauchelements (3) mit Hilfe des Verstellelements (5) einzustellen.

2. Mehrteiliges medizinisches Werkzeug nach Anspruch 1, **dadurch gekennzeichnet, dass** das Gehäuseteil (4) eine Aufnahme (4a) aufweist und dass das distale Ende (3b) des Schlauchelements (3) in der Aufnahme (4a) anordenbar ist beziehungsweise in der Aufnahme (4a) angeordnet ist.

3. Mehrteiliges medizinisches Werkzeug nach Anspruch 1 oder 2, **dadurch gekennzeichnet, dass** das Verstellelement (5) und das distale Ende (2b) der Hohlkanüle (2) über eine Gewindeverbindung (6) funktional wirksam verbindbar beziehungsweise verbunden ist, wobei hierzu das Verstellelement (5) ein Innengewinde (6a) und das distale Ende (2b) beziehungsweise der distale Bereich der Hohlkanüle (2) ein mit dem Innengewinde in Eingriff stehendes Außengewinde (6b) aufweist.

4. Mehrteiliges medizinisches Werkzeug nach Anspruch 3, **dadurch gekennzeichnet, dass** die Gewindeverbindung (6), insbesondere das Innen- und Außengewinde (6a,6b) als ein Feingewinde ausgebildet ist beziehungsweise sind.

5. Mehrteiliges medizinisches Werkzeug nach einem der vorhergehenden Ansprüche, **dadurch gekennzeichnet, dass** von dem distalen Ende (2b) der Hohlkanüle (2) ausgehend zumindest eine sich über zumindest einen Teil der Hohlkanüle (2) axial erstreckende Nut (7) vorgesehen ist.

6. Mehrteiliges medizinisches Werkzeug nach einem der vorhergehenden Ansprüche, **dadurch gekennzeichnet, dass** das Gehäuseteil (4) eine Kavität (8) aufweist, welche zur Aufnahme des distalen Endes (2b) der Hohlkanüle (2) geeignet ist.

7. Mehrteiliges medizinisches Werkzeug nach einem der vorhergehenden Ansprüche, **dadurch gekennzeichnet, dass** sich die Kavität (8) über die gesamte Länge des Gehäuseteils (4) erstreckt und/oder innerhalb der Kavität (8) mindestens ein Fortsatz (4b) ausgebildet ist.

8. Mehrteiliges medizinisches Werkzeug nach einem der vorhergehenden Ansprüche, **dadurch gekennzeichnet, dass** die Kavität (8) als eine Keilnabenbuchse ausgeführt ist oder in die Kavität (8) eine Keilnabenbuchse eingesetzt ist.

9. Mehrteiliges medizinisches Werkzeug nach einem der vorhergehenden Ansprüche, **dadurch gekennzeichnet, dass** der Fortsatz (4b) und/oder das Profil der Keilnabenbuchse in die zumindest eine Nut (7) der Hohlkanüle (2) funktional wirksam eingreift.

10. Mehrteiliges medizinisches Werkzeug nach einem der vorhergehenden Ansprüche, **dadurch gekennzeichnet, dass** das distale Ende (2b) der Hohlkanüle (2) in der Kavität (8) des Gehäuseteils (4), insbesondere in der Keilnabenbuchse positioniert ist.

11. Mehrteiliges medizinisches Werkzeug nach einem der vorhergehenden Ansprüche, **dadurch gekennzeichnet, dass** das distale Ende (2b) der Hohlkanüle (2) in der Kavität (8) des Gehäuseteils (4) oder in der Keilnabenbuchse axial verschiebbar ist.

12. Mehrteiliges medizinisches Werkzeug nach einem der vorhergehenden Ansprüche, **dadurch gekennzeichnet, dass** das distale Ende (3b) des Schlauchelements (3) ein Befestigungselement (9) aufweist.

13. Mehrteiliges medizinisches Werkzeug nach einem der vorhergehenden Ansprüche, **dadurch gekennzeichnet, dass** das Befestigungselement (9) vorzugsweise als eine Rändelmutter, besonders bevorzugt als eine Rändelhohlmutter ausgeführt ist.

14. Mehrteiliges medizinisches Werkzeug nach einem der vorhergehenden Ansprüche, **dadurch gekennzeichnet, dass** das distale Ende (3b) des Schlauchelements (3) mittels der Rändelmutter (9) oder der Rändelhohlmutter in der Aufnahme (4a) des Gehäuseteils (4) verdrehsicher einsetzbar ist.

15. Mehrteiliges medizinisches Werkzeug nach einem der vorhergehenden Ansprüche, **dadurch gekennzeichnet, dass** die Winkellage des Schlauchelements (3) einstellbar ist.

16. Mehrteiliges medizinisches Werkzeug nach einem der vorhergehenden Ansprüche, **dadurch gekennzeichnet, dass** das proximale Ende (2a) der Hohlkanüle (2) eine Krümmung aufweist.

17. Mehrteiliges medizinisches Werkzeug nach einem der vorhergehenden Ansprüche, **dadurch gekennzeichnet, dass** das proximale Ende (2a) der Hohlkanüle (2) und/oder das proximale Ende (3a) des Schlauchelements (3) jeweils eine angeschrägte Öffnung (2c, 3c) aufweist.

18. Mehrteiliges medizinisches Werkzeug nach einem der vorgehenden Ansprüche, **dadurch gekennzeichnet, dass** die Hohlkanüle (2) einen im Wesentlichen gradlinigen Verlauf aufweist, insbesondere aber das proximale Ende (2a) beziehungsweise der proximale Bereich der Hohlkanüle (2) gekrümmt und/oder angeschrägt ist.

19. Mehrteiliges medizinisches Werkzeug nach einem der vorgehenden Ansprüche, **dadurch gekennzeichnet, dass** das Schlauchelement (3) aus einem Formgedächtnismaterial oder einem elastischen Material hergestellt ist und eine vorgeformte Biegung und/oder Krümmung aufweist, wobei der Zustand dieser Biegung und/oder Krümmung außerhalb der Hohlkanüle (2) entsprechend reversibel ausbildbar ist.

20. Mehrteiliges medizinisches Werkzeug nach einem der vorgehenden Ansprüche, **dadurch gekennzeichnet, dass** das Formgedächtnismaterial eine Ni-Ti-Legierung aufweist und/oder das Schlauchelement (3) ein superelastisches Material aufweist beziehungsweise umfasst.

## Claims

1. A multi-part medical tool (1) for intracorporeal injection of a fluid (16), in particular for positioning a liquid and/or viscous collagen in a human joint, in particular in a hip joint (11) or in a shoulder joint, with at least one hollow cannula (2), with at least one flexible tube element (3) arrangeable in the hollow cannula (2), with at least one housing part (4) and with at least one adjusting element (5), wherein the hollow cannula (2) and the tube element (3) each have a proximal end (2a, 3a) and a distal end (2b, 3b), wherein the hollow cannula (2) - in the arranged state of the tube element (3) - at least partially envelops the tube element (3) on a sheath length (M) and the proximal end (3a) of the tube element (3) forms an at least partially curved course outside the sheath length (M), wherein the hollow cannula (2) is displaceably and/or movably arranged relative to the tube element (3), wherein the adjusting element (5) is functionally effective connectable and/or connected to the hollow cannula (2), and wherein the hollow cannula (2) is movable and/or displaceable relative to the tube element (3) by actuation of the adjusting element (5) and the effective sheath length (M) is settable, **characterised in that** the adjusting element (5) is mounted rotatably about the axis of rotation of the hollow cannula (2) in the area of the housing part (4) and braces itself axially on the proximal end face of the housing part (4), so that an axial feed or reverse feed of the hollow cannula (2) can be generated by rotating the adjusting element (5) via a thread, to change the effective sheath length (M) of the hollow cannula (2) and to adjust the curvature and/or length of the exposed proximal end (2a, 3a) of the tube element (3) with the aid of the adjusting element (5).

2. The multi-part medical tool of claim 1, **characterised in that** the housing part (4) has a receiver (4a) and **in that** the distal end (3b) of the tube element (3) is arrangeable in the receiver (4a) or is arranged in the receiver (4a).

3. The multi-part medical tool of claim 1 or 2, **characterised in that** the adjusting element (5) and the distal end (2b) of the hollow cannula (2) are functionally effective connectable or connected via a threaded connection (6), wherein therefor the adjusting element (5) has an internal thread (6a) and the distal end (2b) or the distal area of the hollow cannula (2) has an external thread (6b) engaging with the internal thread.

4. The multi-part medical tool of claim 3, **characterised in that** the threaded connection (6), in particular the internal and external threads (6a, 6b), is or are configured as a fine thread.

5. The multi-part medical tool of any one of the preceding claims, **characterised in that**, starting from the distal end (2b) of the hollow cannula (2), at least one groove (7) extending axially over at least a part of the hollow cannula (2) is provided.

6. The multi-part medical tool of any one of the preceding claims, **characterised in that** the housing part (4) has a cavity (8) which is suitable for receiving the distal end (2b) of the hollow cannula (2).

7. The multi-part medical tool of any one of the preceding claims, **characterised in that** the cavity (8) extends over the entire length of the housing part (4) and/or at least one extension (4b) is configured within the cavity (8).

8. The multi-part medical tool of any one of the preceding claims, **characterised in that** the cavity (8) is designed as a splined hub bushing or a splined hub bushing is inserted into the cavity (8).

9. The multi-part medical tool of any one of the preceding claims, **characterised in that** the extension (4b) and/or the profile of the splined hub bushing functionally effective engages in the at least one groove (7) of the hollow cannula (2).

10. The multi-part medical tool of any one of the preceding claims, **characterised in that** the distal end (2b) of the hollow cannula (2) is positioned in the cavity (8) of the housing part (4), in particular in the splined hub bushing.

11. The multi-part medical tool of any one of the preceding claims, **characterised in that** the distal end (2b) of the hollow cannula (2) is axially displaceable in the cavity (8) of the housing part (4) or in the splined hub bushing.

12. The multi-part medical tool of any one of the preceding claims, **characterised in that** the distal end (3b) of the tube element (3) has a fastening element (9).

13. The multi-part medical tool of any one of the preceding claims, **characterised in that** the fastening element (9) is preferably designed as a knurled nut, particularly preferably as a knurled hollow nut.

14. The multi-part medical tool of any one of the preceding claims, **characterised in that** the distal end (3b) of the tube element (3) is insertable into the receiver (4a) of the housing part (4) in a rotation-resistant manner by means of the knurled nut (9) or the knurled hollow nut.

15. The multi-part medical tool of any one of the preceding claims, **characterised in that** the angular position of the tube element (3) is settable.

16. The multi-part medical tool of any one of the preceding claims, **characterised in that** the proximal end (2a) of the hollow cannula (2) has a curvature.

17. The multi-part medical tool of any one of the preceding claims, **characterised in that** the proximal end (2a) of the hollow cannula (2) and/or the proximal end (3a) of the tube element (3) each has a tapered opening (2c, 3c).

18. The multi-part medical tool of any one of the preceding claims, **characterised in that** the hollow cannula (2) has a substantially straight course, but in particular the proximal end (2a) or the proximal area of the hollow cannula (2) is curved and/or tapered.

19. The multi-part medical tool of any one of the preceding claims, **characterised in that** the tube element (3) is produced from a shape memory material or an elastic material and has a preformed bend and/or curvature, wherein the state of this bend and/or curvature outside the hollow cannula (2) is correspondingly reversibly formable.

20. The multi-part medical tool of any one of the preceding claims, **characterised in that** the shape memory material has a Ni-Ti alloy and/or the tube element (3) has or comprises a superelastic material.

## Revendications

1. Un outil médical (1) en plusieurs parties pour l'injection intracorporelle d'un fluide (16), en particulier pour le positionnement d'un collagène liquide et/ou visqueux dans une jointure humaine, en particulier dans une jointure de la hanche (11) ou dans une jointure de l'épaule, avec au moins une canule creuse (2), avec au moins un élément de tube flexible (3) agençable dans la canule creuse (2), avec au moins une partie de boîtier (4) et avec au moins un élément d'ajustement (5), dans lequel la canule creuse (2) et l'élément de tube (3) ont chacun une extrémité proximale (2a, 3a) et une extrémité distale (2b, 3b), dans lequel la canule creuse (2) - dans l'état agencé de l'élément de tube (3) - enveloppe au moins partiellement l'élément de tube (3) sur une longueur de gaine (M) et l'extrémité proximale (3a) de l'élément de tube (3) forme un cours au moins partiellement incurvé à l'extérieur de la longueur de gaine (M), dans lequel la canule creuse (2) est agencée de manière déplaçable et/ou mobile par rapport à l'élément de tube (3), dans lequel l'élément d'ajustement (5) est fonctionnellement efficace connectable et/ou connecté à la canule creuse (2), et dans lequel la canule creuse (2) est mobile et/ou déplaçable par rapport à l'élément de tube (3) par l'actionnement de l'élément d'ajustement (5) et la longueur efficace de la gaine (M) est réglable, **caractérisé en ce que** l'élément d'ajustement (5) est monté rotatif autour de l'axe de rotation de la canule creuse (2) dans la zone de la partie de boîtier (4) et s'appuie axialement sur la face d'extrémité proximale de la partie de boîtier (4), de manière à pouvoir générer une avance axiale ou une avance inverse de la canule creuse (2) en faisant pivoter l'élément d'ajustement (5) via un filetage, pour modifier la longueur efficace de la gaine (M) de la canule creuse (2) et pour ajuster la curvature et/ou la longueur de l'extrémité proximale exposée (2a, 3a) de l'élément de tube (3) à l'aide de l'élément d'ajustement (5).

2. L'outil médical en plusieurs parties de la revendication 1, **caractérisé en ce que** la partie de boîtier (4) a un récepteur (4a) et **en ce que** l'extrémité distale (3b) de l'élément de tube (3) peut être agencée dans le récepteur (4a) ou est agencée dans le récepteur (4a).

3. L'outil médical en plusieurs parties de la revendication 1 ou 2, **caractérisé en ce que** l'élément d'ajustement (5) et l'extrémité distale (2b) de la canule creuse (2) sont fonctionnellement efficaces connectables ou connectés via une connexion filetée (6), dans laquelle l'élément d'ajustement (5) a un filetage interne (6a) et l'extrémité distale (2b) ou la zone distale de la canule creuse (2) a un filetage externe (6b) s'engageant dans le filetage interne.

4. L'outil médical en plusieurs parties de la revendication 3, **caractérisé en ce que** la connexion filetée (6), en particulier les filetages interne et externe (6a, 6b), est ou sont configurés comme un filetage fin.

5. L'outil médical en plusieurs parties de l'une quelconque des revendications précédentes, **caractérisé en ce que**, à partir de l'extrémité distale (2b) de la canule creuse (2), au moins une rainure (7) s'étendant axialement sur au moins une partie de la canule creuse (2) est fournie.

6. L'outil médical en plusieurs parties de l'une quelconque des revendications précédentes, **caractérisé en ce que** la partie de boîtier (4) a une cavité (8) qui est apte à recevoir l'extrémité distale (2b) de la canule creuse (2).

7. L'outil médical en plusieurs parties de l'une quelconque des revendications précédentes, **caractérisé en ce que** la cavité (8) s'étend sur toute la longueur de la partie de boîtier (4) et/ou au moins une extension (4b) est configurée à l'intérieur de la cavité (8).

8. L'outil médical en plusieurs parties de l'une quelconque des revendications précédentes, **caractérisé en ce que** la cavité (8) est conçue comme une douille à moyeu cannelé ou une douille à moyeu cannelé est insérée dans la cavité (8).

9. L'outil médical en plusieurs parties de l'une quelconque des revendications précédentes, **caractérisé en ce que** l'extension (4b) et/ou le profil de la douille à moyeu cannelé s'engage fonctionnellement efficace dans la au moins une rainure (7) de la canule creuse (2).

10. L'outil médical en plusieurs parties de l'une quelconque des revendications précédentes, **caractérisé en ce que** l'extrémité distale (2b) de la canule creuse (2) est positionnée dans la cavité (8) de la partie de boîtier (4), en particulier dans la douille à moyeu cannelé.

11. L'outil médical en plusieurs parties de l'une quelconque des revendications précédentes, **caractérisé en ce que** l'extrémité distale (2b) de la canule creuse (2) est déplaçable axialement dans la cavité (8) de la partie de boîtier (4) ou dans la douille à moyeu cannelé.

12. L'outil médical en plusieurs parties de l'une quelconque des revendications précédentes, **caractérisé en ce que** l'extrémité distale (3b) de l'élément de tube (3) a un élément de fixation (9).

13. L'outil médical en plusieurs parties de l'une quelconque des revendications précédentes, **caractérisé en ce que** l'élément de fixation (9) est préférablement conçu comme un écrou moleté, en particulier préférablement comme un écrou creux moleté.

14. L'outil médical en plusieurs parties de l'une quelconque des revendications précédentes, **caractérisé en ce que** l'extrémité distale (3b) de l'élément de tube (3) peut être insérée dans le récepteur (4a) de la partie de boîtier (4) de manière à être résistante au pivotement au moyen de l'écrou moleté (9) ou de l'écrou creux moleté.

15. L'outil médical en plusieurs parties de l'une quelconque des revendications précédentes, **caractérisé en ce que** la position angulaire de l'élément de tube (3) est réglable.

16. L'outil médical en plusieurs parties de l'une quelconque des revendications précédentes, **caractérisé en ce que** l'extrémité proximale (2a) de la canule creuse (2) a une curvature.

17. L'outil médical en plusieurs parties de l'une quelconque des revendications précédentes, **caractérisé en ce que** l'extrémité proximale (2a) de la canule creuse (2) et/ou l'extrémité proximale (3a) de l'élément de tube (3) ont chacune une ouverture effilée (2c, 3c).

18. L'outil médical en plusieurs parties de l'une quelconque des revendications précédentes, **caractérisé en ce que** la canule creuse (2) a un cours substantiellement rectiligne, mais en particulier l'extrémité proximale (2a) ou la zone proximale de la canule creuse (2) est incurvé et/ou effilée.

19. L'outil médical en plusieurs parties de l'une quelconque des revendications précédentes, **caractérisé en ce que** l'élément de tube (3) est produit à partir d'un matériau à mémoire de forme ou d'un matériau élastique et a une courbure et/ou une curvature préformée, dans lequel l'état de cette courbure et/ou de cette curvature à l'extérieur de la canule creuse (2) peut être formé de manière correspondamment réversible.

20. L'outil médical en plusieurs parties de l'une quelconque des revendications précédentes, **caractérisé en ce que** le matériau à mémoire de forme a un alliage Ni-Ti et/ou l'élément de tube (3) a ou comprend un matériau superélastique.
